# EUROPÄISCHE PATENTSCHRIFT

(11) **EP 1 429 722 B1**
(45) Veröffentlichungstag und Bekanntmachung des Hinweises auf die Patenterteilung: **05.12.2007**
(21) Anmeldenummer: 02754974.0
(22) Anmeldetag: 03.08.2002
(51) Int. Cl.: A61K 9/00, A61K 9/14

(54) **AUFSTREUVERFAHREN ZUR HERSTELLUNG VON PULVERFORMULIERUNGEN**
SPRAYING METHOD FOR PRODUCING POWDER FORMULATIONS
PROCEDE DE SAUPOUDRAGE POUR LA PRODUCTION DE FORMULATIONS EN POUDRE

(30) Priorität: 23.08.2001 DE 10141377
(43) Veröffentlichungstag der Anmeldung: 23.06.2004
(60) Teilanmeldung: 07116314.1
(73) Patentinhaber: Boehringer Ingelheim Pharma GmbH & Co.KG, 55216 Ingelheim am Rhein (DE)
(72) Erfinder: BOECK, Georg, 55122 Mainz (DE); WALZ, Michael, 55411 Bingen am Rhein (DE)
(86) Internationale Anmeldenummer: PCT/EP2002/008690
(87) Internationale Veröffentlichungsnummer: WO 2003/017970

(56) Entgegenhaltungen:
- WO-A-02/30389
- WO-A-02/30390
- WO-A-96/02231
- FR-M- 8 142
- US-A- 5 478 578
- US-B1- 6 221 390

## Beschreibung

Die Erfindung betrifft ein neues Verfahren zur Herstellung pulverförmiger Zubereitungen für die Inhalation.

### Hintergrund der Erfindung

Bei der Behandlung einer Vielzahl von Erkrankungen, insbesondere bei der Behandlung von Atemwegserkrankungen bietet sich die inhalative Applikation des Wirkstoffs an. Neben der inhalativen Applikation therapeutisch wirksamer Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt. FR 8142 M offenbart Inhalationspulver, in denen der Wirkstoff mit Hilfsstoff verdünnt ist.

In der Pulvermischtechnologie werden üblicherweise Mischverfahren eingesetzt, die auf der Verdünnungsmethode basieren. Hierbei wird der gesamte Wirkstoff vorgelegt und anschließend mit Hilfsstoff beispielsweise in Verhältnissen von 1:1, 1:2 oder 1:4 versetzt und gemischt. Zu den so erhaltenen Mischungen wird dann erneut Hilfsstoff in vergleichbarem Verhältnis zugesetzt. Dieses Vorgehen wird üblicherweise solange wiederholt, bis der ganze Hilfsstoffanteil untergemischt ist. Problematisch an einer solchen Vorgehensweise sind die daraus unter Umständen resultierenden Homogenitätsprobleme. Diese treten insbesondere bei Mischungen auf, bei denen die Substanzen ein stark unterschiedliches Teilchengrößenspektrum aufweisen. Besonders bemerkbar wird dies zudem bei Pulvermischungen, bei denen die Substanz mit der kleineren Teilchengrößenverteilung, der Wirkstoff, nur einen sehr geringen Mengenanteil an der Gesamtpulvermenge aufweist.

Es ist daher Aufgabe der vorliegenden Erfindung, ein Verfahren bereitzustellen, welches die Herstellung von Inhalationspulvern erlaubt, die durch ein hohes Maß an Homogenität im Sinne der Gehaltsgleichförmigkeit gekennzeichnet sind.

### Detaillierte Beschreibung der Erfindung

Überraschenderweise wurde gefunden, daß die eingangs genannte Aufgabe durch ein Verfahren gelöst wird, in dem die Substanz mit der kleineren Teilchengrößenverteilung mittels eines Aufstreuverfahrens auf die Substanz mit der gröberen Teilchengrößenverteilung aufgezogen wird.

Die Erfindung betrifft dementsprechend ein Verfahren zur Herstellung von Pulvermischungen für die Inhalation, dadurch gekennzeichnet, daß zu einem in Bewegung gehaltenen Pulverbett der Substanz mit der größeren Teilchengrößenverteilung die Substanz mit der kleineren Teilchengrößenverteilung über eine geeignete Fördereinreichtung kontinuierlich zudosiert wird, wobei die Mischung mittels eines Zwangsmischers erzeugt und das in Bewegung gehaltene Pulverbett mittels eines Zerhackers verwirbelt wird, wobei die Substanz mit der kleineren Teilchengrößenverteilung ein Wirkstoff mit einer mittleren Teilchengröße von 0,5 bis 10 µm und die Substanz mit der größeren Teilchengrößenverteilung ein Hilfsstoff mit einer mittleren Teilchengröße von 10 bis 100 µm darstellt und wobei der Gehalt der Substanz mit der kleineren Teilchengrößenverteilung bei weniger als 5% liegt.

Im Rahmen der vorliegenden Erfindung repräsentiert, die Substanz mit der kleineren Teilchengrößenverteilung, die sehr fein gemahlen und in einem sehr geringen Massenanteil in der resultierenden Pulverformulierung vorliegt, den Wirkstoff. Im Rahmen der vorliegenden Erfindung repräsentiert, die Substanz mit der größeren Teilchengrößenverteilung, die grob gemahlen und in einem grossen Massenanteil in der resultierenden Pulverformulierung vorliegt, den Hilfsstoff.

Das Pulverbett kann beispielsweise mittels eines Rotors in Bewegung gehalten werden. Je nach apparativer Ausgestaltung kann dieser Rotor bereits Bestandteil des zur Anwendung gelangenden Zwangsmischers sein. Unter einem Zwangsmischer ist ein feststehender Behälter mit sich bewegenden Mischwerkzeugen zu verstehen.

Bevorzugt erfolgt die Zugabe der Komponenten der Pulvermischung im Rahmen des erfindungsgemäßen Verfahrens über eine geeignete Siebeinrichtung, vorzugsweise über einen Siebgranulator mit einer Maschenweite von 0,1 bis 2 mm, besonders bevorzugt 0,3 bis 1 mm, höchst bevorzugt 0,3 bis 0,6 mm.

Gegebenenfalls wird zur Durchführung des erfindungsgemäßen Verfahrens nicht die gesamte Menge an Hilfsstoff bzw. Hilfsstoffmischung, sondern lediglich etwa 85-99%, vorzugsweise etwa 95-98% der Hilfsstoffgesamtmenge, in der Mischapparatur vorgelegt. Dies kann sich insofern als vorteilhaft erweisen, als dann nach kontinuierlicher Zugabe des Wirkstoffs (der Substanz mit kleinerer Teilchengrößenverteilung) mittels der verbleibenden Hilfsstoffmenge (1-15%, vorzugsweise 2-5% der Hilfsstoffgesamtmenge) gegebenenfalls noch in der Siebeinrichtung anhaftender Wirkstoff mit in die Mischeinheit befördert werden kann.

Die vorliegende Erfindung betrifft insbesondere ein Verfahren zur Herstellung von Inhalationspulvern enthaltend weniger als 5%, bevorzugt weniger als 2%, besonders bevorzugt weniger als 1 % Wirkstoff im Gemisch mit einem physiologisch verträglichen Hilfsstoff. Erfindungsgemäß bevorzugt ist ein Verfahren zur Herstellung von Inhalationspulvern, in denen 0,04 bis 0,8%, besonders bevorzugt 0,08 bis 0,64%, besonders bevorzugt 0,16 bis 0,4% Wirkstoff im Gemisch mit einem physiologisch unbedenklichen Hilfsstoff enthalten sind.

Der verwendete Wirkstoff weist erfindungsgemäß eine mittlere Teilchengröße von 0,5 bis 10 µm, vorzugsweise von 1 bis 6 µm, besonders bevorzugt von 2 bis 5 µm auf. Der in das erfindungsgemäße Verfahren einsetzbare Hilfsstoff weist eine mittlere Teilchengröße von 10 bis 100 µm, bevorzugt 15 bis 80 µm, besonders bevorzugt 17 bis 50 µm auf. Erfindungsgemäß besonders bevorzugt sind Verfahren zur Herstellung von Inhalationspulvern, in denen der Hilfsstoff eine mittlere Teilchengröße von 20-30 µm aufweist.

Gegebenenfalls kann der Hilfsstoff auch aus einem Gemisch von gröberem Hilfsstoff mit einer mittleren Teilchengröße von 15 bis 80µm und feinerem Hilfststoff mit einer mittleren Teilchengröße von 1 bis 9 µm bestehen, wobei der Anteil von feinerem Hilfsstoff an der Gesamthilfsstoffmenge 1 bis 20 % betragen kann. Enthalten die mittels des erfindungsgemäßen Verfahrens herstellbaren Inhalationspulver ein Gemisch aus gröberen und feineren Hilfsstofffraktionen, so ist erfindungsgemäß die Herstellung solcher Inhalationspulver bevorzugt, in denen der gröbere Hilfsstoff eine mittlere Teilchengröße von 17 bis 50 µm, besonders bevorzugt von 20 bis 30 µm und der feinere Hilfststoff eine mittlere Teilchengröße von 2 bis 8 µm, besonders bevorzugt von 3 bis 7 µm aufweist. Dabei wird unter der mittleren Teilchengröße im hier verwendeten Sinne der 50 %-Wert aus der Volumenverteilung gemessen mit einem Laserdiffraktometer nach der Trockendispersionsmethode verstanden. Im Fall einer Hilfsstoffmischung aus gröberen und feineren Hilfsstoffanteilen, sind erfindungsgemäß solche Verfahren bevorzugt, mit denen Inhalationspulver erhalten werden, bei denen der Anteil von feinerem Hilfsstoff an der Gesamthilfsstoffmenge 3 bis 15 %, besonders bevorzugt 5 bis 10 % beträgt.

Bei den im Rahmen der vorliegenden Erfindung genannten prozentualen Angaben, handelt es sich stets um Gewichtsprozent.

Soll als Hilfsstoff eines der vorstehend genannten Gemische von gröberem Hilfsstoff mit feinerem Hilfsstoff zum Einsatz gelangen, kann die Hilfsstoffmischung ebenfalls gemäß dem erfindungsgemäßen Verfahren erhalten werden.
In diesem Fall wird zu einem in Bewegung gehaltenen Pulverbett des Hilfsstoffs mit der größeren Teilchengrößenverteilung der Hilfsstoff mit der kleineren Teilchengrößenverteilung über eine geeignete Fördereinreichtung kontinuierlich zudosiert.

Soll als Hilfsstoff eines der vorstehend genannten Gemische von gröberem Hilfsstoff mit feinerem Hilfsstoff zum Einsatz gelangen, kann die Hilfsstoffmischung alternativ zu vorstehend beschriebenem Prozess mittels eines Schichtmischverfahrens erhalten werden. Dieses Schichtmischverfahren ist dadurch gekennzeichnet, daß N+m etwa gleichgroße Portionen des Hilfsstoffs mit größerer Teilchengrößenverteilung und N gleichgroße Portionen des Hilfsstoffs mit kleinerer Teilchengrößenverteilung abwechselnd schichtweise in ein geeignetes Mischgefäß gegeben werden und nach vollständiger Zugabe die 2N+m Schichten der beiden Komponenten mittels eines geeigneten Mischers gemischt werden, wobei zunächst die Zugabe einer Portion des Hilfsstoffs mit größerer Teilchengröße erfolgt, wobei N eine ganze Zahl >0, vorzugsweise >5 ist und wobei m 0 oder 1 bedeutet.
Bevorzugt erfolgt die schichtweise Zugabe der einzelnen Hilfsstofffraktionen über eine geeignete Siebeinrichtung. Gegebenenfalls kann die gesamte Hilfsstoffmischung nach beendetem Mischvorgang noch einem oder mehreren weiteren Siebvorgängen unterworfen werden. Prinzipiell ist erfindungsgemäß bevorzugt, wenn N wenigstens 10 oder mehr, besonders bevorzugt 20 oder mehr, ferner bevorzugt 30 oder mehr beträgt.
Die Zahl m kann 0 oder 1 bedeuten. Steht m für 0 ist die letzte Fraktion, die schichtweise in die geeignete Mischapparatur zugegeben, vorzugsweise eingesiebt wird, die letzte Portion des Hilfsstoffs mit kleinerer Teilchengrößenverteilung. Steht m für die Zahl 1, wird als letzte Fraktion, die schichtweise in die geeignete Mischapparatur zugegeben, vorzugsweise eingesiebt wird, die letzte Portion des Hilfsstoffs mit größerer Teilchengrößenverteilung zugegeben. Die Zugabe der beiden Hilfsstoffkomponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die erste Fraktion der N+m Portionen des gröberen Hilfsstoffs vorgelegt und anschließend die erste Portion der N Portionen des feineren Hilfsstoffanteils in den Mischbehälter eingebracht. Die Zugabe der beiden Komponenten erfolgt abwechselnd durch schichtweises Einsieben der beiden Komponenten.
Nach Herstellung der Hilfsstoffmischung erfolgt aus dieser und dem gewünschten Wirkstoff die Herstellung des Inhaltionspulvers unter Anwendung des erfindungsgemäßen Verfahrens.

Dementsprechend betrifft ein weiterer Aspekt der vorliegenden Erfindung ein Verfahren zur Herstellung von Pulvermischungen, dadurch gekennzeichnet, daß zu einem in Bewegung gehaltenen Pulverbett einer Hilfsstoffmischung bestehend aus einem zuvor erhaltenen Gemisch aus einer Hilfsstofffraktion mit gröberer Teilchengrößenverteilung und einer Hilfsstofffraktion mit kleinerer Teilchengrößenverteilung die Substanz mit der kleineren Teilchengrößenverteilung (der Wirkstoff) über eine geeignete Fördereinrichtung kontinuierlich zudosiert wird.

Die mittels des erfindungsgemäßen Herstellverfahrens erhältlichen Inhalationspulver können generell alle Wirkstoffe enthalten, deren Applikation aus therapeutischer Sicht auf inhalativem Wege sinnvoll erscheint. Bevorzugt gelangen hierbei solche Wirkstoffe zur Anwendung, die beispielsweise ausgewählt sind aus der Gruppe bestehend aus Betamimetika, Anticholinergika, Corticosteroiden und Dopaminagonisten.

Als Betamimetika gelangen hierbei vorzugsweise Verbindungen zur Anwendung, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, Mabuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroky-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-*tert*.-butylamino)ethanol und 1-(4-Ethoxycarbonylamino-3-cyano-5-fluorophenyl)-2-(*tert.*-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe zur Anwendung, die ausgewählt sind aus der Gruppe bestehend aus Fenoterol, Formoterol, Salmeterol, Mabuterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.
Erfindungsgemäß bevorzugt sind die Säureadditionssalze der Betamimetika ausgewählt aus der Gruppe bestehend aus Hydrochlorid, Hydrobromid, Sulfat, Phosphat, Fumarat Methansulfonat und Xinafoat. Besonders bevorzugt sind die Salze im Falle des Salmeterols ausgewählt aus Hydrochlorid, Sulfat und Xinafoat, von denen die Sulfate und Xinafoate besonders bevorzugt sind. Erfindungsgemäß von herausragender Bedeutung sind Salmeterol x ½ H₂SO₄ und Salmeterolxinafoat. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Als Anticholinergika gelangen in den erfindungsgemäßen Verfahren bevorzugt Salze zur Anwendung, die ausgewählt sind aus der Gruppe bestehend aus Tiotropiumsalzen, Oxitropiumsalzen und Ipratropiumsalzen, besonders bevorzugt sind dabei Tiotropium- und Ipratropiumsalze. In den vorstehend genannten Salzen stellen die Kationen Tiotropium, Oxitropium und Ipratropium die pharmakologisch wirksamen Bestandteile dar. Unter den im Rahmen der vorliegenden Erfindung einsetzbaren Salzen sind die Verbindungen zu verstehen, die neben Tiotropium, Oxitropium oder Ipratropium als Gegenion (Anion) Chlorid, Bromid, Iodid, Sulfat, Methansulfonat oder para-Toluolsulfonat enthalten. Im Rahmen der vorliegenden Erfindung sind von allen Salzen der vorstehend genannten Anticholinergika das Methansulfonat, Chlorid, Bromid oder Iodid bevorzugt, wobei dem Methansulfonat oder dem Bromid besondere Bedeutung zukommt. Von erfindungsgemäß herausragender Bedeutung sind die Anticholinergika ausgewählt aus der Gruppe bestehend aus Tiotropiumbromid, Oxitropiumbromid und Ipratropiumbromid. Besonders bevorzugt ist das Tiotropiumbromid. Vorstehend genannte Anticholinergika können gegebenenfalls in Form ihrer Solvate oder Hydrate vorliegen. Im Falle des Tiotropiumbromids kommt beispielsweise dem Tiotropiumbromidmonohydrat eine erfindungsgemäß besondere Bedeutung zu.

Im Rahmen der vorliegenden Erfindung werden unter Corticosteroiden Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Das erfindungsgemäße Verfahren zur Herstellung von Pulvermischungen für die Inhalation kann zur Herstellung von Inhalationspulvern Verwendung finden, die einen oder mehrere der vorstehend genannten Wirkstoffe enthalten. Sollen beispielsweise Inhalationspulver hergestellt werden, deren pharmazeutisch aktive Bestandteile aus zwei verschiedenen Wirkstoffen bestehen, so läßt sich dies mittels des erfindungsgemäßen Verfahrens beispielsweise dadurch gewährleisten, daß zunächst zu einem in Bewegung gehaltenen Pulverbett des Hilfststoffs oder der Hilfsstoffmischung der erste Wirkstoff kontinuierlich zudosiert wird und anschließend zu dieser Pulvermischung in analoger Weise die kontinuierliche Zudosierung des zweiten Wirkstoffs erfolgt. Soll das erfindungsgemäße Verfahren zur Herstellung von Inhalationspulvern dienen, die beispielsweise zwei Wirkstoff enthalten, so seien an dieser Stelle als mögliche Wirkstoffkombinationen die von beispielsweise einem der vorstehend genannten Anticholinergika mit einem der vorstehend genannten Corticosteroide oder die von einem der vorstehend genannten Anticholinergika mit einem der vorstehend genannten Betamimetika als bevorzugt genannt.

Als physiologisch unbedenkliche Hilfsstoffe, die im Rahmen der erfindungsgemäßen Herstellverfahren zur Anwendung gelangen können, seien beispielsweise genannt Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciulcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Inhaltionspulver, die gemäß des erfindungsgemäßen Herstellverfahrens erhalten werden können, zeichnen sich durch ein außergewöhnlich hohes Maß an Homogenität im Sinne der Gehaltsgleichförmigkeit aus. Diese liegt in einem Bereich von < 8% , bevorzugt < 6% , besonders bevorzugt < 4%. Die mittels des erfindungsgemäßen Verfahrens herstellbaren Inhalationspulver weisen gegebenenfalls gar Homogenitäten im Sinne der Einzeldosierungsgenauigkeit auf, die <3%, gegebenenfalls <2% beträgt.

Die mittels des erfindungsgemäßen Verfahrens erhältlichen Inhalationspulver können beispielsweise mittels Inhalatoren appliziert werden, die eine einzelne Dosis aus einem Vorrat mittels einer Meßkammer (z.B. gemäß US 4570630A) oder über andere apparative Vorrichtungen (z.B. gemäß DE 36 25 685 A) dosieren. Vorzugsweise werden die mittels des erfindungsgemäßen Verfahrens erhältlichen Inhalationspulver allerdings in Kapseln abgefüllt (zu sogenannten Inhaletten), die in Inhalatoren wie beispielsweise in der WO 94/28958 beschrieben, zur Anwendung gelangen. Soll das mittels des erfindungsgemäßen Verfahrens erhältliche Inhalationspulver im Sinne der vorstehend gennannten bevorzugten Anwendung in Kapseln (Inhaletten) abgefüllt werden, bieten sich Füllmengen von 3 bis 10 mg, bevorzugt von 4 bis 6 mg Inhalationspulver pro Kapsel an, wobei diese Füllmenge in hohem Maße von der Wahl des verwendeten Wirkstoffs abhängig sein können. Im Falle des Wirkstoffs Tiotropiumbromid enthalten die Kapseln bei den vorstehend genannten Füllmengen zwischen 1,2 und 80 µg Tiotropiumkation. Bei einer für Tiotropiumbromid bevorzugten Füllmenge von 4 bis 6 mg Inhalationspulver pro Kapsel sind zwischen 1,6 und 48 µg, bevorzugt zwischen 3,2 und 38,4 µg, besonders bevorzugt zwischen 6,4 und 24 µg Tiotropium pro Kapsel enthalten. Ein Gehalt von beispielsweise 18 µg Tiotropium entspricht dabei einem Gehalt von etwa 21,7 µg Tiotropiumbromid.

Folglich enthalten Kapseln mit einer Füllmenge von 3 bis 10 mg Inhaltionspulver erfindungsgemäß bevorzugt zwischen 1,4 und 96,3 µg Tiotropiumbromid. Bei einer bevorzugten Füllmenge von 4 bis 6 mg Inhalationspulver pro Kapsel sind zwischen 1,9 und 57,8 µg, bevorzugt zwischen 3,9 und 46,2µg, besonders bevorzugt zwischen 7,7 und 28,9 µg Tiotropiumbromid pro Kapsel enthalten. Ein Gehalt von beispielsweise 21,7 µg Tiotropiumbromid entspricht dabei einem Gehalt von etwa 22,5 µg Tiotropiumbromid-monohydrat.

Folglich enthalten Kapseln mit einer Füllmenge von 3 bis 10 mg Inhalationspulver bevorzugt zwischen 1,5 und 100 µg Tiotropiumbromid-monohydrat. Bei einer bevorzugten Füllmenge von 4 bis 6 mg Inhalationspulver pro Kapsel sind zwischen 2 und 60 µg, bevorzugt zwischen 4 und 48 µg, besonders bevorzugt zwischen 8 und 30 µg Tiotropiumbromid-monohydrat pro Kapsel enthalten.

In den nachfolgenden Beispielen wird eine mögliche Vorgehensweise zur Durchführung des erfindungsgemäßen Verfahrens am Beispiel einer Pulvermischung enthaltend Tiotropiumbromidmonohydrat beschrieben. Die direkte Übertragbarkeit dieses bespielhaft erläuterten Verfahrens zur Herstellung von Inhalationspulvern, die einen oder mehrere der vorstehend genannten anderen Wirkstoffe enthalten, ist für den Fachmann ersichtlich. Dementsprechend dienen die folgenden Beispiele lediglich einer weitergehenden Erläuterung der vorliegenden Erfindung.

### Ausgangsmaterialien

In den nachfolgenden Beispielen wird als gröberer Hilfsstoff Lactose-Monohydrat (200M) verwendet. Dieser kann beispielsweise von der Firma DMV International, 5460 Veghel/NL unter der Produktbezeichnung Pharmatose 200M bezogen werden.

In den nachfolgenden Beispielen wird als feinerer Hilfsstoff Lactose-Monohydrat (5µ) verwendet. Dieser kann durch gängige Verfahren (Mikronisieren) aus Lactose-Monohydrat 200M erhalten werden. Lactose-Monohydrat 200M kann beispielsweise von der Firma DMV International, 5460 Veghel/NL unter der Produktbezeichnung Pharmatose 200M bezogen werden.

### Darstellung von Tiotropiumbromid-monohydrat:

In einem geeigneten Reaktionsgefäß werden in 25,7 kg Wasser und 15,0 kg Tiotropiumbromid, welches wie in der EP 418 716 A1 offenbart, hergestellt werden kann, eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-monohydrat (86 % d. Th.)

Das so erhaltene kristalline Tiotropiumbromid-monohydrat wird nach bekannten Verfahren mikronisiert, um den Wirkstoff in Form der mittleren Teilchengröße bereitzustellen, die den erfindungsgemäßen Spezifikationen entspricht.

Im Sinne der vorliegenden Erfindung ist unter mittlerer Teilchengröße der Wert in µm zu verstehen, an dem 50% der Teilchen aus der Volumenverteilung eine kleinere oder die gleiche Partikelgröße besitzen im Vergleich zum angegebenen Wert. Zur Bestimmung der Summenverteilung der Partikelgrößenverteilung wird als Meßmethode Laserdiffraktion/Trockendispergierung angewendet.

Nachfolgend wird beschrieben, wie die Bestimmung der mittleren Teilchengröße der verschiedenen Bestandteile der erfindungsgemäßen Formulierung erfolgen kann.

### A) Partikelgrößenbestimmung von feinteiliger Lactose:

### Meßgerät und Einstellungen:

Die Bedienung der Geräte erfolgt in Übereinstimmung mit den Bedienungsanleitungen des Herstellers.

| | |
|---|---|
| Meßgerät: | HELOS Laser-Beugungs-Spektrometer, (SympaTec) |
| Dispergiereinheit: | RODOS Trockendispergierer mit Saugtrichter, (SympaTec) |
| Probenmenge: | ab 100 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 1 bis 15 sek. (im Fall von 100 mg) |
| Brennweite: | 100 mm (Meßbereich: 0,9 - 175 µm) |
| Meßzeit: | ca. 15 s (im Fall von 100 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

### Probenvorbereitung / Produktzufuhr:

Mind. 100 mg der Prüfsubstanz werden auf einem Kartenblatt eingewogen.
Mit einem weiteren Kartenblatt werden alle größeren Agglomerate zerstoßen. Das Pulver wird dann auf der vorderen Hälfte der Schwingrinne (ab ca. 1 cm vom vorderen Rand) fein verteilt aufgestreut. Nach dem Start der Messung wird die Frequenz der Schwingrinne von ca. 40 % bis 100 % (gegen Ende der Messung) variiert. Die Zeit, in der jeweils die gesamte Probe zugeführt wird beträgt 10 bis 15 sek.

### B) Partikelgrößenbestimmung von Tiotropiumbromidmonohydrat, mikronisiert:

### Meßgerät und Einstellungen:

Die Bedienung der Geräte erfolgte in Übereinstimmung mit den Bedienungsanleitungen des Herstellers.

| | |
|---|---|
| Meßgerät: | Laser-Beugungs-Spektrometer (HELOS), Sympatec |
| Dispergiereinheit: | Trockendispergierer RODOS mit **Saugtrichter,Sympatec** |
| Probenmenge: | 50 mg - 400 mg |
| Produktzufuhr: | Schwingrinne Vibri, Fa. Sympatec |
| Frequenz d. Vibrationsrinne: | 40 bis 100 % ansteigend |
| Dauer der Probenzufuhr: | 15 bis 25 sek. (im Fall von 200 mg) |
| Brennweite: | 100 mm (Meßbereich: 0,9 - 175 µm) |
| Meßzeit: | ca. 15 s (im Fall von 200 mg) |
| Zykluszeit: | 20 ms |
| Start/Stop bei: | 1 % auf Kanal 28 |
| Dispergiergas: | Druckluft |
| Druck: | 3 bar |
| Unterdruck: | maximal |
| Auswertemodus: | HRLD |

### Probenvorbereitung / Produktzufuhr:

ca. 200 mg der Prüfsubstanz werden auf einem Kartenblatt eingewogen. Mit einem weiteren Kartenblatt werden alle größeren Agglomerate zerstoßen.
Das Pulver wird dann auf der vorderen Hälfte der Schwingrinne (ab ca. 1 cm vom vorderen Rand) fein verteilt aufgestreut.
Nach dem Start der Messung wird die Frequenz der Schwingrinne von ca. 40 % bis 100 % (gegen Ende der Messung) variiert. Die Zufuhr der Probe soll möglicht kontinuierlich sein. Die Produktmenge darf aber auch nicht zu groß sein damit eine ausreichende Dispergierung erreicht wird. Die Zeit, in der jeweils die gesamte Probe zugeführt wird beträgt für 200 mg z. B. ca. 15 bis 25 sek.

### C) Partikelgrößenbestimmung von Laktose 200M :

### Meßgerät und Einstellungen

Die Bedienung der Geräte erfolgte in Übereinstimmung mit den Bedienungsanleitungen des Herstellers.

| | |
|---|---|
| Meßgerät: | Laser-Beugungs-Spektrometer (HELOS), |
| | Sympatec |
| Dispergiereinheit: | Trockendispergierer RODOS mit Saugtrichter, Sympatec |
| Probenmenge: | 500 mg |
| Produktzufuhr: | Vibrationsrinne Typ VIBRI , Sympatec |
| Frequenz d. Vibrationsrinne: | 18 bis 100 % ansteigend |
| Brennweite (1): | 200 mm (Meßbereich: 1.8 - 350 µm) |
| Brennweite (2): | 500 mm (Meßbereich: 4.5 - 875 µm) |
| Meßzeit/Wartezeit: | 10 s |
| Zykluszeit: | 10 ms |
| Start/Stop bei: | 1 % auf Kanal 19 |
| Druck: | 3 bar |
| Unterdruck: | maximal |
| Auswertemodus: | HRLD |

### Probenvorbereitung / Produktzufuhr:

Ca. 500 mg der.Prüfsubstanz werden auf einem Kartenblatt eingewogen. Mit einem weiteren Kartenblatt werden alle größeren Agglomerate zerstoßen. Das Pulver wird in der Trichter der Vibrationsrinne überführt. Es wird ein Abstand von 1.2 bis 1.4 mm zwischen Vibrationsrinne und Trichter eingestellt. Nach dem Start der.Messung wird die Amplitudeneinstellung der Schwingrinne von 0 auf 40 % gesteigert bis sich ein kontinuierlicher Produktfluß einstellt. Danach wird auf eine Amplitude von ca. 18% reduziert. Gegen Ende der Messung wird die Amplitude auf 100% gesteigert.

### Apparatives

Zur Herstellung der erfindungsgemäßen Inhalationspulver können beispielsweise die folgenden Maschinen und Geräte Verwendung finden:
Zwangsmischer: Diosna, Typ P100; Hersteller: Firma Dierks und Söhne, D-49009 Osnabrück.
Mischbehälter bzw. Pulvermischer: Rhönradmischer 200 L; Typ: DFW80N-4; Hersteller: Firma Engelsmann, D-67059 Ludwigshafen.
Siebgranulator: Quadro Comil; Typ: 197-S; Hersteller: Firma Joisten & Kettenbaum, D-51429 Bergisch-Gladbach.
Fördereinrichtung: KtronSoder; Typ T20 bzw. T35; Hersteller: Firma K-Tron Soder GmbH, D 6460 Gelnhausen.
Fördereinrichtung (für Kleinmengen/speziell Wirkstoff): Gericke; Typ GMD60/2; Hersteller: Firma Gericke GmbH, D 78239 Rielasingen.

### Beispiel 1:

Die Durchführung des nachfolgend beschriebenen Schritts 1.1 zur Darstellung einer Hilfsstoffmischung kann gegebenenfalls auch entfallen. Wird eine Pulvermischung angestrebt, die neben dem Wirkstoff lediglich Hilfsstoff einer einheitlichen gröberen Teilchengrößenverteilung enthält, kann direkt gemäß Schritt 1.2 verfahren werden.

### 1.1: Hilfsstoffmischung:

Als gröbere Hilfsstoffkomponente werden 31,82 kg Lactose Monohydrat für Inhalationszwecke (200M) eingesetzt. Als feinere Hilfsstoffkomponente werden 1,68 kg Lactose Monohydrat (5µm) eingesetzt. In den daraus erhaltenen 33,5 kg Hilfsstoffmischung beträgt der Anteil der feineren Hilfsstoffkomponente 5%.

Über einen geeigneten Siebgranulator mit einem Sieb mit einer Maschenweite von 0,5 mm werden in einen geeigneten Mischbehälter ca. 0,8 bis 1,2 kg Lactose Monohydrat für Inhalationszwecke (200M) vorgelegt. Anschließend werden abwechselnd Lactose Monohydrat (5µm) in Portionen von ca. 0,05 bis 0,07 kg und Lactose Monohydrat für Inhalationszwecke (200M) in Portionen von 0,8 bis 1,2 kg schichtweise eingesiebt. Lactose Monohydrat für Inhalationszwecke (200M) und Lactose Monohydrat (5µm) werden in 31 bzw. in 30 Schichten (Toleranz: ± 6 Schichten) zugegeben.

Die eingesiebten Bestandteile werden anschließend mit einem Freifallmischer gemischt (Mischen: 900 Umdrehungen)

### 1.2: wirkstoffhaltige Pulvermischung:

Zur Herstellung der Endmischung werden 32,87 kg der Hilfsstoffmischung (1.1) und 0,13 kg Mikronisiertes Tiotropiumbromid-monohydrat eingesetzt. In dem daraus erhaltenen 33,0 kg Inhalationspulver beträgt der Wirkstoffanteil 0.4 %.

In einem geeigneten Zwangsmischer werden 32 kg der gemäß 1.1 erhaltenen Hilfsstoffmischung vorgelegt. Der Zwangsmischer wird in Betrieb genommen und der Zerhacker zugeschaltet. Zu dem in Bewegung gehaltenen Hilfstoffpulverbett wird über eine geeignete Fördereinrichtung 0,13 kg mikronisiertes Tiotropiumbromid-monohydrat langsam und kontinuierlich zudosiert. Der Arzneistoff kann bei Bedarf zusätzlich über einen geeigneten Siebgranulator mit einem Sieb mit einer Maschenweite von 0,5 mm gegeben werden. Anschließend wird die restliche Hilfsstoffmischung über die Fördereinrichtung und ggf. den Siebgranulator in den laufenden Zwangsmischer zugeführt. Nach Zuführung der Mischungsbestandteile wird noch für weitere 2 Minuten gemischt. Beim Auftreten von Belägen an der Mischerwandung werden diese mittels eines Kunststoffschabers abgeschabt. Danach wird erneut für 2 Minuten gemischt.

### Beispiel 2:

Mit der nach Beispiel 1 erhaltenen Mischung werden Inhalationskapseln (Inhaletten) der folgenden Zusammensetzung erhalten:

| | |
|---|---|
| Tiotropiumbromid-monohydrat: | 0,0225 mg |
| Lactose Monohydrat (200 M): | 5,2025 mg |
| Lactose Monohydrat (5 µm): | 0,2750 mg |
| Hartgelatinekapsel: | 49,0 mg |
| Total: | 54,5 mg |

### Beispiel 3:

Inhalationskapsel der Zusammensetzung:

| | |
|---|---|
| Tiotropiumbromid-monohydrat: | 0,0225 mg |
| Lactose Monohydrat (200 M): | 4,9275 mg |
| Lactose Monohydrat (5 µm): | 0,5500 mg |
| Hartgelatinekapsel: | 49,0 mq |
| Total: | 54,5 mg |

Das zur Herstellung der Kapsel erforderliche Inhalationspulver wurde in Analogie zu Beispiel 1 erhalten.

### Beispiel 4:

lnhalationskapsel der Zusammensetzung:

| | |
|---|---|
| Tiotropiumbromid-monohydrat: | 0,0225 mg |
| Lactose Monohydrat (200 M): | 5,2025 mg |
| Lactose Monohydrat (5 µm): | 0,2750 mg |
| Polyethylenkapsel: | 100,0 mg |
| Total: | 105,50 mg |

Das zur Herstellung der Kapsel erforderliche Inhalationspulver wurde in Analogie zu Beispiel 1 erhalten.

## Patentansprüche

1. Verfahren zur Herstellung von Pulvermischungen für die Inhalation, **dadurch gekennzeichnet, daß** zu einem in Bewegung gehaltenen Pulverbett der Substanz mit der größeren Teilchengrößenverteilung die Substanz mit der kleineren Teilchengrößenverteilung über eine geeignete Fördereinrichtung kontinuierlich zudosiert wird, wobei die Mischung mittels eines Zwangsmischers erzeugt und das in Bewegung gehaltene Pulverbett mittels eines Zerhackers verwirbelt wird, wobei die Substanz mit der kleineren Teilchengrößenverteilung ein Wirkstoff mit einer mittleren Teilchengröße von 0,5 bis 10 µm und die Substanz mit der größeren Teilchengrößenverteilung ein Hilfsstoff mit einer mittleren Teilchengröße von 10 bis 100 µm darstellt und wobei der Gehalt der Substanz mit der kleineren Teilchengrößenverteilung bei weniger als 5 Gewichts-% liegt.

2. Verfahren nach Anspruch 1, **dadurch gekennzeichnet, daß** die Zugabe der Komponenten der Pulvermischung über eine geeignete Siebeinrichtung erfolgt.

3. Verfahren nach einem der Ansprüche 1 bis 2, **dadurch gekennzeichnet, daß** Pulvermischungen für die Inhalation erzeugt werden, in der die Substanz mit der kleineren Teilchengrößenverteilung ausgewählt ist aus der Gruppe bestehend aus Betamimetika, Anticholinergika, Corticosteroiden und Dopaminagonisten.

## Claims

1. Process for preparing powder mixtures for inhalation, **characterised in that** the substance with the smaller particle size distribution is metered continuously, through a suitable feed device, onto a moving bed of the powdered substance with the coarser particle size distribution, the mixture being produced by means of a forced-action mixer and the moving bed of powder being fluidised by means of a vibrator, wherein the substance with the smaller particle size distribution is an active substance having a mean particle size of from 0.5 to 10 µm and the substance with the coarser particle size distribution is an excipient having a mean particle size of from 10 to 100 µm, and the content of the substance with the smaller particle size distribution is less than 5 wt.%.

2. Process according to claim 1, **characterised in that** the components of the powder mixture are added by means of a suitable screening device.

3. Process according to one of claims 1 to 2, **characterised in that** powder mixtures for inhalation are produced wherein the substance with the smaller particle size distribution is selected from among the betamimetics, anticholinergics, corticosteroids and dopamine agonists.

## Revendications

1. Procédé de production de mélanges de poudres pour l'inhalation **caractérisé en ce que** la substance à répartition granulométrique plus fine est ajoutée en continu par le biais d'un dispositif de transport approprié à un lit de poudre maintenu en mouvement de la substance à répartition granulométrique plus grossière, où le mélange est produit au moyen d'un malaxeur à mélange forcé et le lit de poudre maintenu en mouvement est amené à tourbillonner au moyen d'un hacheur, où la substance à répartition granulométrique plus fine représente un principe actif ayant une granulométrie moyenne de 0,5 à 10 µm et la substance à répartition granulométrique plus grossière représente un adjuvant ayant une granulométrie moyenne de 10 à 100 µm et où la teneur de la substance à répartition granulométrique plus fine est inférieure à 5 % en masse.

2. Procédé selon la revendication 1 **caractérisé en ce que** l'addition des composants du mélange de poudres a lieu par le biais d'un dispositif de tamisage approprié.

3. Procédé selon l'une des revendications 1 à 2 **caractérisé en ce que** des mélanges de poudres pour l'inhalation dans lesquels la substance à répartition granulométrique plus fine est choisie dans le groupe consistant en les bêtamimétiques, les anticholinergiques, les corticostéroïdes et les agonistes de dopamine sont produits.
